# EUROPEAN PATENT APPLICATION

(11) **EP 3 560 504 A1**
(43) Date of publication of application: **30.10.2019**
(21) Application number: 18305501.1
(22) Date of filing: 23.04.2018
(51) Int. Cl.: A61K 35/28, A61K 35/51, A61P 17/02

(54) **CELLS DIFFERENTIATED FROM MESENCHYMAL STEM CELLS FOR THE TREATMENT OF DERMAL LESIONS**

(71) Applicant: Commissariat à l'Energie Atomique et aux Energies Alternatives, 75015 Paris (FR); Universidade Nacional De La Plata, Pcia de Buenos Aires (AR); Buero, Guillermo Tomás, C1112AAJ Ciudad Autonoma de Buenos Aires (AR)
(72) Inventor: BUERO, Tomas Guillermo., C1112AAJ Ciudad Autonoma de Buenos Aires (AR); GARCIA, Marcella, 1900 LA PLATA Pcia de Buenos Aires (AR); CAROSELLA, Edgardo D., 75475 PARIS CEDEX 10 (FR)
(74) Representative: Gevers & Orès

(57) **Abstract**

The invention pertains to a population of isolated cells which is homogenous for the phenotype CD44⁺, CD73⁺, CD29⁺, CD105⁺, CD90⁺, HLA-G⁺ and HLA-DR⁻, or compositions comprising thereof, for use as a medicament, as well as to methods for preparing such cells.

## Description

The invention pertains to the field of therapeutic treatment of dermal lesions, based on cells differentiated from mesenchymal stem cells.

### PRIOR ART

Normally, injury to the skin sets cicatrization, which involves a complex cascade of events ultimately resulting in wound healing, namely the formation of the neodermis and re-epithelialization. Acute skin wounds result from some form of trauma, and undergo a repair process that, when it is orderly and timely, lead to a benign scar. Failure of this process, because the wound area and/or depth exceed the patient's ability to heal, may lead to an undesirable scar or a chronic or nonhealing wound. The ability to heal seems to diminish with age-related decreased strength and elasticity of skin, decreased blood flow to the extremities due to sedentary lifestyle, smoking, and other factors.

Inadequate and/or incomplete wound healing, however, can lead to significant individual morbidity including a profound reduction in quality of life. Sores can become seriously infected, gangrenous and in some cases require amputation. Some pathologies, such as skin ulcers and more particularly leg ulcers, are more likely to be subject to incomplete wound healing, The leg ulcer is described as the spontaneous or accidental tissue absence in a varicose leg, for more than fifteen days, with no signs of healing despite conventional treatment. Several tissues may be involved in this process, dermis, epidermis, subcutaneous tissue, muscle and even bone may contribute to the development of the disease.

For patients who have chronic wounds that are difficult to heal, basic medical and surgical care is essential, but is not always sufficient. Current therapies for wound healing and in particular leg ulcer, include removal of unhealthy tissue, use of growth factors, hyperbaric (high-pressure) oxygen treatment, advanced wound dressings such as elastic bandages, Unna boot and hydrocolloid dressings, skin grafts, and surgery, such as varicose vein surgery. However, none of these approaches regenerate skin appendages (e.g. hair follicles, sweat glands).

In recent years, advances have been made in accelerating healing of chronic wounds and ulcers. In particular, the use of stem cell-based therapies has been suggested, based on evidence suggesting that bone marrow-derived stem cells are also recruited into the wound site and may have an important contribution to the wound healing process, in addition to blood-borne immunocompetent cells.

Hence, MSCs have received considerable attention for modulating wound repair (Bajada et al., J Tissue Eng Regen Med.; 2(4): 169-83), 2008). MSCs have been examined in skin repair and regeneration after various acute and chronic skin injuries, such as acute incisional and excisional wounds, diabetic skin ulcers, radiation burns, and thermal burns (Wu *et al*.; 25(10):2648-59, 2007; Lataillade et al., Regen Med.; 2(5):785-94, 2007; Vojtassak et al. Neuro Endocrinol Lett.; 27(Suppl 2): 134-7), 2006).

While these results with MSCs are impressive, the treatment approach generally requires that the MSCs be cultured in sufficient numbers for topical application; this may not be a major issue for small chronic wounds, but may become very impractical when treating large wounds. In addition, all of the above treatment rely on the utilization of autologous cells, which limits their application and further implies complications for the patients: first, the bloody and painful method of extraction for obtaining bone marrow MSCs, second, the necessary waiting time, from the collection of the original sample to the *in vitro* expansion of the cells, to obtain a sufficient number of them for the treatment. In this context, there is thus a need for alternative or improved therapeutic options for the treatment of epidermal lesions, and in particular with chronic lesions.

### DESCRIPTION

The inventors have set up a method that enables the differentiation of mesenchymal stem cells into a population of cells which is homogenous for the phenotype CD44⁺, CD73⁺, CD29⁺, CD105⁺, CD90⁺, HLA-G⁺ and HLA-DR⁻.

The population of cells possess immunomodulation capacity (as supported by the immunomodulation capacity test detailed in the experimental part), and further is particularly efficient in the treatment of dermic lesions such as ulcers, usually difficult to treat. The population of cells of the invention promotes wound healing, improving the rapidity of cicatrization and enabling complete recovery, that is to say regeneration of the tissue. These properties are of particular interest in the treatment of dermic lesions, in particular ulcers, such as leg ulcer, and chronic fistulas.

Although not bound by theory, it is likely that the beneficial properties of the population of cells according to the invention resides , at least in art, in the cells expressing HLA-G. Indeed, the HLA-G molecule is a non-classical class I HLA molecule which has effective immunosuppressive properties, and has been shown to play an essential role in feto-maternal tolerance and allogenic tissue graft tolerance. Two cellular membrane receptors interact whit HLA-G: ILT2 and ILT4. The ILT2 receptor is expressed on the monocytes/macrophages, dendritic cells, B, and T lymphocytes particularly in the cytotoxic T subset and natural Killer cells. The ILT4 receptor is expressed on myeloid cells: monocytes, dendritic and APC cells. The interaction between HLA-G and these receptors confers a strong immunosuppressive activity, capable to inhibit the immune response at different levels.

In addition, the cells resulting from the method of differentiation of the invention do not express HLA-DR, and as a result are not limited to autologous therapy and can be used in heterologous therapy, that is to say applied to patients having a different HLA-DR than the HLA-DR of the donor of mesenchymal stem cells.

The invention pertains to a population of isolated cells which is homogenous for the phenotype CD44⁺, CD73⁺, CD29⁺, CD105⁺, CD90⁺, HLA-G⁺ and HLA-DR⁻.

In the context of the invention, the terms **"CD29"** refer to the human cell-surface protein CD29, also known as Integrin subunit beta-1, which is a product of the human ITGB1 gene (Entrez gene ID: 3688). In the context of the invention, a cell having a CD29⁺ phenotype is a cell expressing on its surface the CD29 protein as defined above.

Expression of a protein on the surface of a cell can easily be assessed using established techniques known in the art, such as for instance flow cytometry.

In the context of the invention, the terms **"CD44"** refer to the human cell-surface glycoprotein CD44, which is a product of the human CD44 gene (Entrez gene ID: 960). In the context of the invention, a cell having a CD44⁺ phenotype is a cell expressing on its surface the CD44 glycoprotein as defined above.

In the context of the invention, the terms **"CD73"** refer to the human cell-surface antigen CD73, also known as ecto-5'-nucleotidase or 5'-nucleotidase (5'-NT), which is a product of the human NT5E gene (Entrez gene ID: 4907). In the context of the invention, a cell having a CD73⁺ phenotype is a cell expressing on its surface the CD73 antigen as defined above.

In the context of the invention, the terms **"CD90"** refer to the human N-glycosylated, glycophosphatidylinositol (GPI) cell surface protein CD105, also known as Thy-1, which is a product of the human THY1 gene (Entrez gene ID: 7070). In the context of the invention, a cell having a CD90⁺ phenotype is a cell expressing on its surface the CD90 protein as defined above.

In the context of the invention, the terms **"CD105"** refer to the human cell-surface glycoprotein CD105, also known as Endoglin, which is a product of the human ENG gene (Entrez gene ID: 2022).In the context of the invention, a cell having a CD105⁺ phenotype is a cell expressing on its surface the CD105 protein as defined above.

In the context of the invention, the terms **"HLA-G"** refer to any of the non-classical class I HLA antigen (immune tolerant protein) cell surface proteins HLA-G, expressed by the human HLA-G gene (Entrez gene ID: 3135). The human HLA-G gene encodes both the membrane-bounds proteins HLA-G1, HLA-G2, HLA-G3, HLA-G4 and the soluble proteins HLA-G5, HLA-G6, HLA-G7. Accordingly, in the context of the invention, the terms "HLA-G" encompass the human proteins HLA-G1, HLA-G2, HLA-G3, HLA-G4. In the context of the invention, a cell having a HLA-G⁺ phenotype is a cell expressing on its surface any of the human proteins HLA-G1, HLA-G2, HLA-G3, HLA-G4.as defined above.

In the context of the invention, the terms **"HLA-DR"** refer to the human MHC class II cell surface receptors HLA-DR, a family of αβ heterodimerd formed of an α-chain which does not vary, paired with any of the β-chain encoded by the genes of any of the loci HLA-DRB1, HLA-DRB3, HLA-DRB4, and HLA-DRB5. The α-chain is thus encoded by the HLA-DRA locus (Entrez gene ID: 3122). The β-chain may result from the expression of HLA-DRB1 locus (Entrez gene ID: 3123), the HLA-DRB3 locus (Entrez gene ID: 3125), the HLA-DRB4 locus (Entrez gene ID: 3126), or the HLA-DRB5 locus (Entrez gene ID: 3127). In the context of the invention, a cell having a HLA-DR⁻ phenotype is a cell which does not express on its surface any of the proteins encoded by the HLA-DRA, the HLA-DRB1, the HLA-DRB3, the HLA-DRB4, or the HLA-DRB5 locus.

In the context of the invention, a population of cells is considered has "homogenous for the phenotype CD44⁺, CD73⁺, CD29⁺, CD105⁺, CD90⁺, HLA-G⁺ and HLA-DR⁻" when said population of cells consists essentially of cells having the phenotype CD44⁺, CD73⁺, CD29⁺, CD105⁺, CD90⁺, HLA-G⁺ and HLA-DR⁻.

Preferably, the population of cells of the invention is characterized in that:
- at least 90%, preferably at least 95%, yet preferably at least 99% of the cells within the population have the phenotype CD44⁺,
- at least 90%, preferably at least 95%, yet preferably at least 99% of the cells within the population have the phenotype CD73⁺,
- at least 90%, preferably at least 95%, yet preferably at least 99% of the cells within the population have the phenotype CD29⁺,
- at least 90%, preferably at least 95%, yet preferably at least 99% of the cells within the population have the phenotype CD105⁺,
- at least 90%, preferably at least 95%, yet preferably at least 99% of the cells within the population have the phenotype CD90⁺,
- at least 90%, preferably at least 95%, yet preferably at least 99% of the cells within the population have the phenotype HLA-G⁺ and
- all of the cells within the population have the phenotype HLA-DR⁻.

More preferably, the population of cells of the invention is characterized in that at least 90%, preferably at least 95%, yet preferably at least 99% of the cells within the population have the phenotype CD44⁺, CD73⁺, CD29⁺, CD105⁺, CD90⁺, HLA-G⁺, and all of the cells have the phenotype HLA-DR⁻.

Yet preferably, the population of cells of the invention is characterized in that all of the cells within the population have the phenotype CD44⁺, CD73⁺, CD29⁺, CD105⁺, CD90⁺, HLA-G⁺ and HLA-DR⁻.

Preferably, the population of cells according to the invention is a population of pluripotent cells.

Preferably, the population of cells according to the invention is derived, advantageously by differentiation, from a population of MSCs, more preferably from a population of MSCs obtained from Wharton's jelly, yet preferably from a population of MSCs obtained from human umbilical cord's Wharton's jelly.

In the context of the invention, the term **"mesenchymal stem cells" or "MSCs"** refer to multipotent stem cells, able to differentiate into a variety of cell types, present in biological tissues of mesenchyme origin, such as umbilical cord tissue, preferably Wharton's jelly and the umbilical cord blood, bone marrow, adipose tissue, the developing tooth bud of the mandibular third molar, and amniotic fluid.

Preferably, the population of MSCs according to the invention is homogenous for the phenotype CD90⁺, CD73⁺, CD105⁺, CD166⁺, CD172alpha⁺, CD171⁺, CD11b⁻, CD14⁻, CD19⁻, CD34⁻, CD45⁻, CD79A-, CD309⁻ and HLA-DR⁻.

In the context of the invention, the terms **"CD14"** refer to the human membrane-bound protein mCD14, which is a product of the human CD14 gene (Entrez gene ID: 929). In the context of the invention, a cell having a CD14⁻ phenotype is a cell that does not express the CD14 protein as defined above on its surface.

In the context of the invention, the terms **"CD19"** refer to the human membrane-bound protein CD19, which is a product of the human CD19 gene (Entrez gene ID: 930). In the context of the invention, a cell having a CD19⁻ phenotype is a cell that does not express the CD19 protein as defined above on its surface.

In the context of the invention, the terms **"CD34"** refer to the human cell surface glycoprotein CD34, which is encoded by the human CD34 gene (Entrez gene ID: 947). In the context of the invention, a cell having a CD34⁻ phenotype is a cell that does not express the CD34 protein as defined above on its surface.

In the context of the invention, the terms **"CD45"** refer to the human antigen CD45, also known as Protein tyrosine phosphatase, receptor type, C (PTPRC), which is encoded by the human PTPRC gene (Entrez gene ID: 5788). In the context of the invention, a cell having a CD45⁻ phenotype is a cell that does not express the CD45 protein as defined above on its surface.

In the context of the invention, the terms **"CD79A⁻"** refer to alpha chain of the human transmembrane protein CD79, also known as B-cell antigen receptor complex-associated protein alpha chain, which is encoded by the human CD79A gene (Entrez gene ID: 973).

In the context of the invention, a cell having a CD79A⁻ phenotype is a cell that does not express the CD79A protein as defined above on its surface.

In the context of the invention, the terms **"CD166"** refer to the human transmembrane glycoprotein CD166 antigen, which is encoded by the human ALCAM gene (Entrez gene ID: 214). In the context of the invention, a cell having a CD166⁺ phenotype is a cell expressing on its surface the CD166 protein as defined above.

In the context of the invention, the terms **"CD172alpha⁺"** refer to the human Signal regulatory protein alpha (SIRPα), which is encoded by the human SIRPA gene (Entrez gene ID: 140885). In the context of the invention, a cell having a CD172alpha⁺ phenotype is a cell expressing on its surface the CD172alpha⁺ protein as defined above.

In the context of the invention, the terms **"CD309"** refer to the human membrane-bound vascular endothelial growth factor receptor 2, also known as Kinase insert domain receptor (KDR), which is a product of the human KDR gene (Entrez gene ID: 3791). In the context of the invention, a cell having a CD309⁻ phenotype is a cell that does not express the CD309 protein as defined above on its surface.

In the context of the invention, the terms **"CD11b"** refer to the human Integrin alpha M protein, also known as Complement receptor 3 A (CR3A), which is a product of the human KDR gene (Entrez gene ID: 3684). In the context of the invention, a cell having a CD11b⁻ phenotype is a cell that does not express the CD11b protein as defined above on its surface.

In the context of the invention, the term **"MSCs obtained from Wharton's jelly"** refers to a population of MSCs homogenous for the phenotype CD90+, CD73+, CD105+, CD166+, CD172alpha+, CD171+, CD11b-, CD14-, CD19-, CD34-, CD45-, CD79a-, CD309- and HLA-DR-, and isolated from Wharton's jelly.

**Wharton's Jelly,** also referred to in the art as *substantia gelatinea funiculi umbilicalis* is a gelatinous substance derived from extra-embryonic mesoderm, present within the umbilical cord. Wharton's Jelly is mostly comprised of mucopolysaccharides, such as hyaluronic acid and chondroitin sulfate, and has been shown to comprise MSCs, and more particularly MSCs having sternness and immune properties. MSCs harvested from the umbilical cord's Wharton's jelly are expected to exhibit a much more proliferative, immunosuppressive, and even therapeutically active stem cells than those isolated from older, adult tissue sources such as the bone marrow or adipose tissue.

In the context of the invention, the term **"MSCs obtained from the umbilical cord's Wharton's jelly"** refers to a population of MSCs homogenous for the phenotype CD90⁺, CD73⁺, CD105⁺, CD166⁺, CD172alpha⁺, CD171⁺, CD11b⁻, CD14⁻, CD19⁻, CD34⁻, CD45⁻, CD79a⁻, CD309⁻ and HLA-DR⁻, and isolated from the umbilical cord's Wharton's jelly, preferably from umbilical cord's Wharton's jelly of human origin.

In the context of the invention, a population of cells is considered has homogenous for the phenotype CD90⁺, CD73⁺, CD105⁺, CD166⁺, CD172alpha⁺, CD171⁺, CD11B⁻, CD14⁻, CD19⁻, CD34⁻, CD45⁻, CD79A⁻, CD309⁻ and HLA-DR⁻ when:
- at least 90%, preferably at least 95%, yet preferably at least 99% of the cells within the group have the phenotype CD90⁺
- at least 90%, preferably at least 95%, yet preferably at least 99% of the cells within the group have the phenotype CD73⁺,
- at least 90%, preferably at least 95%, yet preferably at least 99% of the cells within the group have the phenotype CD73⁺,
- at least 90%, preferably at least 95%, yet preferably at least 99% of the cells within the group have the phenotype CD105⁺,
- at least 90%, preferably at least 95%, yet preferably at least 99% of the cells within the group have the phenotype CD166⁺,
- at least 90%, preferably at least 95%, yet preferably at least 99% of the cells within the group have the phenotype CD172alpha⁺,
- at least 90%, preferably at least 95%, yet preferably at least 99% of the cells within the group have the phenotype CD171⁺,
- at least 90%, preferably at least 95%, yet preferably at least 99% of the cells within the group have the phenotype CD11B⁻,
- at least 90%, preferably at least 95%, yet preferably at least 99% of the cells within the group have the phenotype CD14⁻,
- at least 90%, preferably at least 95%, yet preferably at least 99% of the cells within the group have the phenotype CD19⁻,
- at least 90%, preferably at least 95%, yet preferably at least 99% of the cells within the group have the phenotype CD34⁻,
- at least 90%, preferably at least 95%, yet preferably at least 99% of the cells within the group have the phenotype CD45⁻,
- at least 90%, preferably at least 95%, yet preferably at least 99% of the cells within the group have the phenotype CD79A⁻,
- at least 90%, preferably at least 95%, yet preferably at least 99% of the cells within the group have the phenotype CD309⁻, and
- at least 90%, preferably at least 95%, yet preferably at least 99% of the cells within the group have the phenotype HLA-DR⁻.

Preferably, in the context of the invention, a population of cells is considered has homogenous for the phenotype CD90⁺, CD73⁺, CD105⁺, CD166⁺, CD172alpha⁺, CD171⁺, CD11B⁻, CD14⁻, CD19⁻, CD34⁻, CD45⁻, CD79A⁻, CD309⁻ and HLA-DR⁻ when at least 90%, preferably at least 95%, yet preferably at least 99% of the cells within the group have the phenotype CD90⁺, CD73⁺, CD105⁺, CD166⁺, CD172alpha⁺, CD171⁺, CD11B⁻, CD14⁻, CD19⁻, CD34⁻, CD45⁻, CD79A⁻, CD309⁻ and HLA-DR⁻.

The population of cells of the invention may conveniently be obtained by implementing the following method of differentiation, using MSCs, preferably of MSCs obtained from Wharton's jelly, more preferably of MSCs obtained from the umbilical cord's Wharton's jelly, yet preferably MSCs obtained from human umbilical cord's Wharton's jelly, as starting material.

The invention further pertains to a method of differentiation, comprising the successive steps of:
a) providing a population of MSCs, more preferably of MSCs obtained from Wharton's jelly, yet preferably of MSCs obtained from human umbilical cord's Wharton's jelly;
b) culturing the cells in an appropriate culture medium;
c) recovering a population of cells which is homogenous for the phenotype CD44⁺, CD73⁺, CD29⁺, CD105⁺, CD90⁺, HLA-G⁺ and HLA-DR⁻.

Regarding step a) of the method of the invention, MSCs cells can easily be isolated according to methods known in the art, from tissues known to contain them such as indicated above. Further, MSCs may be obtained in particular from Wharton's Jelly, by any of the method known in the art. Such methods have been thoroughly disclosed for instance by McElreavey K.D. et al. (Biochem. Soc. Trans.;19:29S, 1991), Kadner A. et al. (Eur. J. Cardio-Thorac. Surg.;25:635-641, 2004), Mitchell K.E. et al. (Stem Cells;21:50-60, 2003), and Kim et al. (Int J Mol Sci.; 14(6): 11692, 2013).

The person skilled in the art may easily verify that the population of MSCs is actually homogenous for the phenotype CD90⁺, CD73⁺, CD105⁺, CD166⁺, CD172alpha⁺, CD171⁺, CD11B⁻, CD14⁻, CD19⁻, CD34⁻, CD45⁻, CD79A⁻, CD309⁻ and HLA-DR⁻, using the methods disclosed above.

With regards to step b) of the method of the invention, it should be construed that the cells are cultured in appropriate conditions of temperature, humidity and atmosphere composition (i.e. O₂ and CO₂ in particular). For instance, the cells may be incubated at 37° in a humid atmosphere, in the presence of 5% of CO₂.

In the context of the invention, the terms **"appropriate culture medium"** refer to any cell culture medium appropriate for the differentiation of MSCs cells. Preferably, the appropriate culture medium comprises platelet lysate, such as described in Luzzani et al. yet preferably, the appropriate culture medium comprises alpha MEM base medium supplemented with 10% of platelet lysate. Preferably, the appropriate culture medium is xeno-free, in other terms all components of the cell culture medium are derived from the same organism, preferably derived from human origin. Advantageously, the appropriate culture medium does not contain products of animal origin other than human.

In step b) of the method of the invention, the cells are cultured up until they form a population of cells which comprises a sufficient number of cells having the phenotype CD44⁺, CD73⁺, CD29⁺, CD105⁺, CD90⁺, HLA-G⁺ and HLA-DR⁻, for instance a population wherein more than 50% of the cells have the phenotype CD44⁺, CD73⁺, CD29⁺, CD105⁺, CD90⁺, HLA-G⁺ and HLA-DR⁻.

Preferably, the cells are cultured up until they form a population of cells which is homogenous for the phenotype CD44⁺, CD73⁺, CD29⁺, CD105⁺, CD90⁺, HLA-G⁺ and HLA-DR.

Preferably, the culture of step b) is performed for at least 14 days, preferably for between 14 and 40 days, yet preferably for between 20 and 35 days.

By the end of step b), the population of cells may already contains contain enough cells having the phenotype CD44⁺, CD73⁺, CD29⁺, CD105⁺, CD90⁺, HLA-G⁺ and HLA-DR⁻, in which case step c) may easily be performed by simply collecting the cultured cells.

Alternatively, in order to recover a population of cells which is homogenous for the phenotype CD44⁺, CD73⁺, CD29⁺, CD105⁺, CD90⁺, HLA-G⁺ and HLA-DR⁻ as defined above, the person skilled in the art may add steps of selecting the cells having the appropriate phenotype.

It has been observed that the population of cells of the invention possess immunomodulation capacity, which, without being bound by theory, is likely to stem from their HLA-G⁺ phenotype. In order to verify the status of the population of cells with regards to this functional feature, the person skilled in the art may add to the method of the invention a further step comprising an immunomodulation capacity test.

Preferably, the method of the invention comprises a further step of assessing that the population of cells from step c) has immunomodulation capacity.

In the context of the invention, the terms **"immunomodulation capacity"** Should be construed as generally understood in the field, i.e. as the ability to suppress the proliferation of lymphocytes, preferably of CD4 and CD8 T lymphocytes. Such capacity can be assayed using methods well known in the art, such as that disclosed in the experimental part. In brief, the population of cells of the invention is co-cultured with PBMCs previously pre-stimulated with concanavalin-A, in a medium appropriate for the culture of lymphocytes, such as RPMI-1640 supplemented with 10% FBS, said medium further comprising interleukin-2. By the end of the co-culture, the proliferation of lymphocytes, preferably of CD4 and CD8 T lymphocytes, is measured and compared with control conditions (i.e. culture of PBMCs in the absence of the cells of the invention). In the context of the invention, a population of cells is considered has having "immunomodulation capacity" when it limits or suppress the proliferation of lymphocytes, preferably of CD4 and CD8 T lymphocytes, in comparison with said control.

Advantageously, the method of the invention comprises a further step of selecting population of cells which has immunomodulation capacity as defined above.

The population of cells according to the invention may either be used directly, preferably for their therapeutic use as detailed thereafter, or advantageously be frozen and stored for later use. Cells may be frozen using usual methods known in the art, and are preferably stored suspended in a medium comprising alpha MEM, platelet Lysate and DMSO, at a temperature of -20°C or less, preferably of -80°C of less. After being defrost for their intended use, the phenotype of the population of cells may be assessed to verify that its matches the definition given above.

Another aspect of the invention relates to a pharmaceutical composition, comprising a population of isolated pluripotent stem cells according to the invention, and, preferably, a pharmaceutically acceptable carrier.

Suitable vehicles or carriers include any pharmaceutically acceptable vehicle such as buffering agents, stabilizing agents, diluents, salts, preservatives, emulsifying agents, sweeteners, etc. The vehicle typically comprises an isotonic aqueous or non-aqueous solution, which may be prepared according to known techniques. Suitable solutions include buffered solutes, such as phosphate buffered solution, chloride solutions, Ringer's solution, and the like.

A further aspect of the invention relates to the population of cells, or compositions comprising thereof according to the invention for use as a medicament.

Preferably, the population of cells or composition comprising thereof according to the invention is for use in the treatment of lesions of biological tissues.

In the context of the invention, the terms "biological tissues" refer to human bodily tissues and preferably epitheliums, preferably the dermis, epidermis, and/or subcutaneous tissue.

Advantageously, the population of cells or composition comprising thereof according to the invention is for improving regeneration of said lesion.

In the context of the invention, the terms **"regeneration"** refers to the formation of new tissue in place of the injured tissue. In particular with reference to the dermis, epidermis, and/or subcutaneous tissue, regeneration involves the formation of new tissue including formation of structures usually present in such tissues such as hair follicles and glands. The term "regeneration" is not equivalent to the term "cicatrization", the latter involving only the replacement of normal tissue with connective tissue without formation of new as hair follicles and glands.

Preferably, the population of cells or composition comprising thereof according to the invention is for use in the treatment of lesions of the epithelium tissue, yet preferably lesions of the dermis, epidermis, and/or subcutaneous tissue. Advantageously, the population of cells or composition comprising thereof according to the invention is for use in the treatment of chronic lesions, preferably chronic lesions of the dermis, epidermis, and/or subcutaneous tissue, yet preferably ulcers.

In the context of the invention, the term **"ulcers"** is to be construed according to the general meaning in the art, that is to say as a sore on the skin or a mucous membrane, accompanied by the disintegration of tissue, occurring as a result of impaired blood circulation, bacterial or viral infections, fungal infections or cancers.

Advantageously, the epidermal lesions are epidermal lesions associated with chronic leg ulcers, epidermal lesions associated with decubitus ulcers, and chronic fistulas.

In the context of the invention, the terms **"chronic leg ulcers"** are to be construed according to the general meaning in the art, that is to say as the spontaneous or accidental tissue lesions in a varicose leg, for more than fifteen days, with no signs of healing despite conventional treatment, said lesions occurring as a result of a deep venous thrombosis.

In the context of the invention, the terms **"decubitus ulcers"** are to be construed according to the general meaning in the art, that is to say as the tissue lesions to the skin and/or underlying tissue that usually occur over a bony prominence as a result of pressure, or pressure in combination with shear and/or friction.

The invention also provides a method for the treatment of a lesions, more preferably the treatment of lesions of the epithelium tissue, yet preferably epidermal lesions, in a subject, comprising: administering to said subject a population of cells or a composition comprising thereof according to the invention.

Preferably, the population of cells or composition comprising thereof according to the invention, is administered in a therapeutically effective amount.

By **"therapeutically effective amount"** it is hereby referred to an amount which is, over time, sufficient to at least improve the disease, in the context of the invention improve the lesion, preferably improve cicatrization and regeneration of the lesion. Typically, said amount can be adjusted by the skilled artisan, depending on the pathological condition, the subject, the duration of treatment, the presence of other active ingredients, in order to obtain the desired therapeutic response.

Preferably the population of cells or composition comprising thereof according to the invention is administered through the parenteral route, preferably via intravenous, intramuscular, subcutaneous or intradermal administration. Yet preferably the population of cells or composition comprising thereof according to the invention is administered is administered through subcutaneous or intradermal administration, advantageously intradermal administration.

Preferably, the population of cells or composition comprising thereof according to the invention, is administered by injection.

As herein defined, the term **"injection"** refers to the continuous administration of liquid or medication in a subject, via intradermal or subcutaneous application, lasting less than 15 minutes. Injection typically involves the administration of said fluid or medication through a needle or a catheter.

The invention also comprises other provisions that will emerge from the following examples of implementation, which may not be construed as limiting the scope of the invention.

### LEGEND OF THE FIGURES

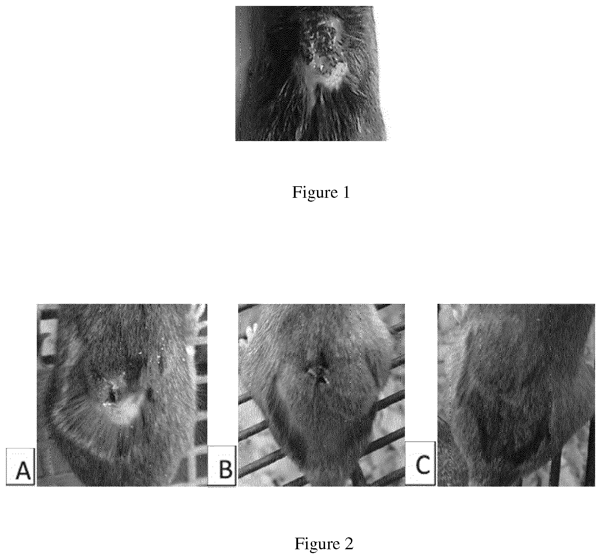

Figure 1 - Picture of the initial lesion obtained using disposable dermatology biopsy punch on mice. This lesion involves epidermis, dermis and cellular subcutaneous tissues. The lesion size is about 0.7 mm of diameter.
Figure 2 - Pictures of the evolution of wound healing. The initial lesion as illustrated in figure 1 was either not treated (group 1), treated with an MDC injection from DMC of murine umbilical cord (group 2) or treated with MDC injection from DMC of human umbilical cord (group 3). One week after initiation of the treatment, pictures were taken for each of the 3 groups. Figure 2. A is representative of group 1, i.e. controls animals without treatment. The lesion began cicatrize but show some cicatricial blood clot and no pilous growth. Figure 2.B is representative of group 2, i.e. mice treated with intradermal injection of DMC from murine umbilical cords on the edges of the injury. Skin regeneration could be observed, with a little cicatricial blood clot and the skin regenerates and formation of new hair follicles. Figure 2.C is representative of the group 3, i.e. mice treated with intradermal injection of DMC from human umbilical cords on the edges of the injury. Skin regeneration could be observed, with formation of new hair follicles.

### EXAMPLES

### EXAMPLE 1

The effect of cells differentiated from mesenchymal cells from Warton's jelly on wound healing was assayed using inmunocompetent mice C3H/S as models.

### Preparation of cells differentiated from mesenchymal cells from Warton's jelly

Mesenchymal stem cells were obtained from human umbilical cords using the following protocol.

Human umbilical cords obtained from discarded placentas are placed in DMEM culture medium. Cords are chopped in 5 mm length items. The cuts are made in the middle part of the length of the umbilical cord. Every portion, about 5 mm long, is sagittally cut in the amniotic membrane and the mesenchymal mucoid connective tissue (Wharton Jelly) is exposed. The mucoid connective perivascular tissue and the umbilical's vessels are extracted. The gelatin face of Wharton jelly is put down in direct contact with the cell culture plate, and cultured in the conditions described in Luzzani et al., that is to say in a culture medium comprising alpha MEM base medium supplemented with 10% of platelet lysate. Plates are incubated at 37° in a humid atmosphere, in the presence of 5% of CO₂. Fresh culture medium is added each 2-3 days. When cells reach confluence, they are passaged, typically using tripsin. MSCs expansion is observed after 10/14 days, and cells are amplified until passage 3. DMC are characterized by surface markers for DMC typical antigen (CD44⁺, CD73⁺, CD29⁺, CD105⁺, CD90⁺, HLA-G⁺, HLA-DR⁻), and cells positive for this phenotype are selected using flow cytometry.

### Immunomodulation capacity test of cells differentiated from mesenchymal cells from Warton's jelly

Lymphocytes proliferation is analyzed by *in vitro* inhibition. Briefly, heparinized blood samples were resuspended in PBS (1:1). Diluted blood was then carefully layered on top of the ficoll (Histopaque-1077, Sigma, US) at a ratio 1.5:1 and centrifuged at 800g for 30 minutes. The buffy coat layer containing mononuclear cells was aspirated, and the PBMCs were then either cultured for immediate use in RPMI-1640 (Gibco, US) supplemented with 10% FBS, or frozen for later use. Before the experiment, an appropriate number of PBMCs were stained with CFSE (Life Technologies, US) and pre-stimulated with concanavalin-A (Sigma, US) for 24 hs. Then, one-day stimulated PBMCs (i.e.e peripheral blood mononuclear cells) were washed with PBS and co-cultered with DMCs for 96 hours in RPMI-1640 supplemented with 10% FBS, 10 ng/ml anti-CD3 antibody (clone UCHT1) and 300 IU/ml interleukin-2 (both from R&D Systems, US). PBMCs where then collected and analyzed by flow cytometry. Unstained, stained but not stimulated and stained stimulated PBMC without DMC were also run as controls. Flow cytometry analyses were performed in a BD Accuri cytometer. A standard protocol for cell staining was used. Briefly, cells were washed with PBS and a single cell suspension was obtained by incubating them with Accutasse. Cells are then washed with PBS plus 0.5% albumin (PBS/Alb), and incubated at room temperature for 30 minutes with the appropriate antibody concentration. Cells were then washed with PBS/Alb and analyzed. At least 5000 events were counted.

The data obtained confirm that DMC generate a profound suppression of the proliferation of CD4 and CD8 T lymphocytes.

Finally, DMC are frost in cryotubes and stored in the Master bank. The cells are washed with PBS, recovered with trypsin. The PBS is replaced with DMEM + 10% BFS to inactivate trypsin. Cells are washed and centrifuged at 1000 G for 5 minutes. The obtained pellets are resuspended in alphaMEM + 20 % platelet lysate + 10 % DMSO. The cryotubes were placed at a final concentration of 1 x 106 DMC in 1 ml.

Cells are defrost and culture for expansion, and passaged (until a maximum of 6 passages).

Cells may be used directly or frost for storage in view of future uses.

### In vivo tests on mice.

Three groups of inmunocompetent mice C3H/S were constituted, each comprising 6 mice.

A lesion was induced on the dorsal part of these mice using a disposable dermatology biopsy punch. Figure 1 illustrates the lesion obtained.

Group 1: Dorsal lesion with no other therapy

Group 2: Dorsal lesion treated with perilesional intradermal injection of 250.000 DMC/0.1 ml PBS, differentiated from MSC from murine umbilical cords.

Group 3: Dorsal lesion treated with perilesional injection of 250.000 DMC/0.1 ml PBS, differentiated from MSC from human umbilical cords.

One week after initiation of the treatment, pictures of the wounds in each groups were taken. These pictures are presented in figure 2.

The evolution of wound healing was monitored until complete epidermal cicatrization and pilosity recovery.

In the group 1, lesions healed after 12 days and pilosity was recovered in 25 days. In the group 2, lesions healed after 9 days and pilosity was recovered in 20 days. In the group 3, lesions healed after 5 days and pilosity was recovered in 10 days. In conclusion, group 3 had fastest lesion recovery, as healing time was reduced by 50% compared to the control.

This invention shows that after intradermal injection of allogenic DMC: no inflammatory reaction was observed (as per direct observation or histological analysis) and lesions of the treated mice from group 3 healed earlier than those of group 1 and 2.

### REFERENCES

Bajada S, Mazakova I, Richardson JB, Ashammakhi N. Updates on stem cells and their applications in regenerative medicine. J Tissue Eng Regen Med. 2008 Jun;2(4):169-83.
Wu Y, Chen L, Scott PG, Tredget EE. Mesenchymal stem cells enhance wound healing through differentiation and angiogenesis. Stem Cells. 2007 Oct;25(10):2648-59.
Lataillade JJ, Doucet C, Bey E, Carsin H, Huet C, Clairand I, et al. New approach to radiation burn treatment by dosimetry-guided surgery combined with autologous mesenchymal stem cell therapy. Regen Med. 2007 Sep;2(5):785-94.
Vojtassak J, Danisovic L, Kubes M, Bakos D, Jarabek L, Ulicna M, et al. Autologous biograft and mesenchymal stem cells in treatment of the diabetic foot. Neuro Endocrinol Lett. 2006 Dec;27(Suppl 2):134-7).
McElreavey K.D., Irvine A.I., Ennis K.T., McLean W.H. Isolation, culture and characterisation of fibroblast-like cells derived from the wharton's jelly portion of human umbilical cord. Biochem. Soc. Trans. 1991;19:29S.
Chacko A.W., Reynolds S.R.M. Architecture of distended and nondistended human umbilical cord tissues, with special reference to the arteries and veins. Contrib. Embryol. 1954;35:135-150.
Kadner A., Hoerstrup S.P., Tracy J., Breymann C., Maurus C.F., Melnitchouk S., Kadner G., Zund G., Turina M. Human umbilical cord cells: A new cell source for cardiovascular tissue engineering. Ann. Thorac. Surg. 2002;74:S1422-S1428.
Kadner A., Zund G., Maurus C., Breymann C., Yakarisik S., Kadner G., Turina M., Hoerstrup S.P. Human umbilical cord cells for cardiovascular tissue engineering: A comparative study. Eur. J. Cardio-Thorac. Surg. 2004;25:635-641.
Kim D-W, Staples M, Shinozuka K, Pantcheva P, Kang S-D, Borlongan CV. Wharton's Jelly-Derived Mesenchymal Stem Cells: Phenotypic Characterization and Optimizing Their Therapeutic Potential for Clinical Applications. International Journal of Molecular Sciences. 2013;14(6):11692-11712.
Luzzani C, Neiman G, Garate X, et al. A therapy-grade protocol for differentiation of pluripotent stem cells into mesenchymal stem cells using platelet lysate as supplement. Stem Cell Research & Therapy. 2015;6(1):6.
Mitchell K.E., Weiss M.L., Mitchell B.M., Martin P., Davis D., Morales L., Helwig B., Beerenstrauch M., Abou-Easa K., Hildreth T., et al. Matrix cells from wharton's jelly form neurons and glia. Stem Cells. 2003;21:50-60.
Romanov Y.A., Svintsitskaya V.A., Smirnov V.N. Searching for alternative sources of postnatal human mesenchymal stem cells: Candidate msc-like cells from umbilical cord. Stem Cells. 2003;21:105-110.
Takechi K., Kuwabara Y., Mizuno M. Ultrastructural and immunohistochemical studies of wharton's jelly umbilical cord cells. Placenta. 1993;14:235-245.

## Claims

1. A population of isolated cells which is homogenous for the phenotype CD44⁺, CD73⁺, CD29⁺, CD105⁺, CD90⁺, HLA-G⁺ and HLA-DR⁻, or compositions comprising thereof, for use as a medicament.

2. A population of isolated cells which is homogenous for the phenotype CD44⁺, CD73⁺, CD29⁺, CD105⁺, CD90⁺, HLA-G⁺ and HLA-DR⁻, or compositions comprising thereof, for use in the treatment of lesions, preferably the treatment of lesions of the epithelium tissue, more preferably epidermal lesions.

3. The population of isolated cells or the composition comprising thereof, for its use according to claim 2, **characterized in that** the epidermal lesions are epidermal lesions associated with chronic leg ulcers, epidermal lesions associated with decubitus ulcers, and chronic fistulas.

4. The population of isolated cells or the composition comprising thereof, for its use according to any of claims 2 or 3, **characterized in that** it is for regenerating the tissue in said of said lesion.

5. The population of isolated cells or the composition comprising thereof, for its use according to any of claims 1 to 4, **characterized in that** said population of isolated cells is derived, advantageously by differentiation, from a population of MSCs, more preferably from a population of MSCs obtained from Wharton's jelly, yet preferably from a population of MSCs obtained from human umbilical cord's Wharton's jelly.

6. The population of isolated cells or the composition comprising thereof, for its use according to any of claims 1 to 4, **characterized in that** said population of isolated cells is obtained by the method of claim 7.

7. A method of differentiation, comprising the successive steps of:
a) providing a population of MSCs, more preferably of MSCs obtained from Wharton's jelly, yet preferably of MSCs obtained from human umbilical cord's Wharton's jelly;
b) culturing the cells in an appropriate culture medium;
c) recovering a population of cells which is homogenous for the phenotype CD44+, CD73⁺, CD29⁺, CD105⁺, CD90⁺, HLA-G⁺ and HLA-DR⁻.

8. A pharmaceutical composition comprising a population of isolated cells which is homogenous for the phenotype CD44⁺, CD73⁺, CD29⁺, CD105⁺, CD90⁺, HLA-G⁺ and HLA-DR⁻, and a pharmaceutically acceptable carrier.

9. A pharmaceutical composition comprising a population of isolated cells which is obtained by the method of claim 7, and a pharmaceutically acceptable carrier.
